# EUROPÄISCHE PATENTANMELDUNG

(11) **EP 3 056 169 A1**
(43) Veröffentlichungstag der Anmeldung: **17.08.2016**
(21) Anmeldenummer: 16000728.2
(22) Anmeldetag: 22.05.2013
(51) Int. Cl.: A61F 2/24

(54) **IMPLANTIERBARE VORRICHTUNG ZUR VERBESSERUNG ODER BEHEBUNG EINER HERZKLAPPENINSUFFIZIENZ**

(30) Priorität: 01.06.2012 DE 102012010798
(62) Teilanmeldung aus: 13729592.9
(71) Anmelder: Universität Duisburg-Essen, 45141 Essen (DE)
(72) Erfinder: Neumann, Till, 44869 Bochum (DE); Erbel, Raimund, 45131 Essen (DE)
(74) Vertreter: Cohausz Hannig Borkowski Wißgott

(57) **Zusammenfassung**

Die Erfindung betrifft eine Implantierbare Vorrichtung zur Verbesserung oder Behebung einer Herzklappeninsuffizienz umfassend einen Verschlußkörper (1), der im Durchgangsbereich einer Herzklappe positionierbar ist, insbesondere im Bereich zwischen einem Atrium (2) und einem Ventrikel (3) des Herzens und der ein oberes, stromaufwärtiges Ende (1a) und ein unteres, stromabwärtiges Ende (1 b) aufweist, wobei an wenigstens einem der Enden (1a, 1 b) des Verschlußkörpers (1) wenigstens ein Anlageband (4) angeordnet ist, das sich von dem Verschlußkörper (1) weg erstreckt und in wenigstens einer Schlaufe zu dem Verschlußkörper (1) zurückgeführt ist und zumindest entlang eines Teils seiner Erstreckung an die innere Herzwand anlegbar ist.

## Beschreibung

Die Erfindung betrifft eine implantierbare Vorrichtung zur Verbesserung oder Behebung einer Herzklappeninsuffizienz umfassend einen Verschlußkörper, der im Durchgangsbereich einer Herzklappe positionierbar ist, insbesondere im Bereich zwischen einem Atrium und einem Ventrikel des Herzens und der ein oberes, stromaufwärtiges Ende und ein unteres, stromabwärtiges Ende aufweist.

Vorrichtungen dieser Art sind im Stand der Technik bekannt, z.B. aus der Publikation US 7,785,366 B2. Die darin beschriebene Vorrichtung weist einen Verschlußkörper auf, der im Durchgangsbereich einer Mitralklappe und somit zwischen dem linken Atrium und linken Ventrikel des Herzens angeordnet wird. Hierfür wird der Verschlußkörper mit einem sich nach unten erstreckenden Ankerelement im Myokard des Herzens befestigt. Das Ankerelement weist eine derart gewählte Länge auf, dass der Verschlußkörper innerhalb der Herzklappe angeordnet, d.h. von dieser umgeben ist. Die hier beschriebene Vorrichtung weist den Nachteil auf, dass zur Befestigung des Verschlußkörpers eine Implantation im Myokard essentiell ist, was eine Infektionsgefahr und eine Gewebeschädigung mit sich bringt. Auch ist der Verschlußkörper gemäß dieser Bauart nur zu einer Seite verankert und kann sich am Ankerende frei um den Implantationsort herum bewegen. Eine Ortsfestigkeit innerhalb der Herzklappe ist somit nicht mit Sicherheit gegeben.

Allgemein sind Verschlußkörper dieser Art einsetzbar, um Herzklappeninsuffizienzen zu beheben oder zumindest zu verbessern, d.h. solche krankhaften Veränderungen oder Fehlbildungen der Herzklappen, die darin resultieren, dass die Herzklappen nicht vollständig schließen, sondern selbst im geschlossenen Zustand ein Spalt zwischen den Klappensegeln verbleibt, durch den Blut entgegen der eigentlichen Flußrichtung fließen kann. Eine Herzklappeninsuffizienz kann daher zu einer Sauerstoff-Unterversorgung, damit zu einer geringeren Belastbarkeit und im schlimmsten Fall zum Tode führen.

Ein Verschlußkörper der eingangs genannten gattungsgemäßen Art ist somit vorgesehen, um den im geschlossenen Zustand einer Herzklappe verbleibenden Spalt auszufüllen und diesen Spalt hierdurch zu verschließen, zumindest jedoch zu verkleinern, so dass eine Herzklappe ihre Funktion im geschlossenen Zustand zurückerhält.

Ein Verschlußkörper der hier eingangs genannten, gattungsgemäßen Art ist nicht auf die zum Stand der Technik genannte Anwendung bei einer Insuffizienz der Mitralklappe beschränkt. Es kann eine Anwendung auch bei anderen Klappen des Herzens vorgesehen sein, z.B. bei der Trikuspidalklappe, Pulmonalklappe, Aortenklappe.

Es ist die Aufgabe der Erfindung eine in das Herz implantierbare Vorrichtung mit einem Verschlußkörper bereit zu stellen, die auf einfache Art und Weise implantierbar ist und im Herzen auf schonende Art positionierbar und befestigbar ist und bevorzugt die Positionierung sicher eingehalten wird.

Diese Aufgabe wird erfindungsgemäß dadurch gelöst, dass bei einer Vorrichtung der eingangs genannten Art an wenigstens einem der Enden des Verschlußkörpers wenigstens ein Anlageband angeordnet ist, das sich von dem Verschlußkörper weg erstreckt und in wenigstens einer Schlaufe zu dem Verschlußkörper zurückgeführt ist und zumindest entlang eines Teils seiner Erstreckung an die innere Herzwand anlegbar ist.

Der wesentliche Kerngedanke bei der Erfindung ist es, dass eine Befestigung, d.h. ortsfeste Positionierung des Verschlußkörpers nicht mehr durch eine Implantation im Myokard erzielt wird, wenngleich eine solche Befestigung in einer Weiterbildung zusätzlich zur erfindungsgemäßen Befestigungsart auch noch vorgesehen sein kann.

Die erfindungsgemäße Befestigung erfolgt dadurch, dass das in wenigstens einer Schlaufe geführte Anlageband an die innere Herzwandung angelegt werden kann und dabei zumindest entlang eines Teils seines Schlaufenumfangs die Herzwand nach einer Implantation kontaktiert. Die wenigstens eine Schlaufe ist somit an der Innenwandung des Herzens entlanggeführt.

Dabei kann es in einer Weiterbildung vorgesehen sein, dass ein Anlageband auf dem Weg seiner Erstreckung aufzweigt in wenigstens zwei Anlagebandarme, die zum Verschlußkörper zurückgeführt sind. Eine solche Aufzweigung kann z.B. an einem Ort maximaler Entfernung des Aufzweigungsortes vom Verschlußkörper erfolgen.

Eine erfindungsgemäße Konstruktion kann auch vorsehen, dass vom Verschlußkörper ausgehend mehrere Anlagebandarme sich auf einem schlaufenförmigen oder bogenförmigen Weg erstrecken und in einer gemeinsamen Verbindungstelle treffen und dort zusammengeführt sind. Eine solche Konstruktion bildet einen Käfig aus mehreren Anlagebandarmen aus, die mit einem der Enden am Verschlußkörper befestigt sind und mit den anderen Enden untereinander.

In bevorzugter Ausführung kann es vorgesehen sein, dass ein Anlageband aus einem federelastischen Material ausgebildet ist, insbesondere einem federelastischen Material aus einer Formgedächtnislegierung ausgebildet ist.

Die federelastische Ausbildung hat den Vorteil, dass eine Anlage des wenigstens einen Anlagebandes an der inneren Herzwandung unter der Kraftbelastung erfolgen kann, die durch eine innere Federkraft erzeugt ist. Z.B. kann die Anlagebandlänge, bzw. Schlaufenlänge oder die Größe eines aus mehreren Anlagebandarmen gebildeten Käfigs größer gewählt sein als das gewählte Atrium oder die gewählte Herzkammer, in der das Anlageband befestigt werden soll.

In einem solchen Fall wird ein Anlageband / Käfig durch die Herzwand gestaucht, was eine der Stauchung entgegen wirkende Federkraft im Anlageband / Käfig erzeugt. Auf diese Weise wird ein guter Kraft- und Formschluß erzielt. Diese Ausführung hat auch den Vorteil, dass durch die Federelastizität die Kontraktion des Herzens durch die erfindungsgemäße Vorrichtung nicht beeinträchtigt wird.

Die Ausbildung des wenigstens einen Anlagebandes aus einem federelastischen Material, insbesondere einer Formgedächtnislegierung, wie z.B. Nitinol, hat den weiteren Vorteil, dass das wenigstens eine Anlageband, z.B. zum Zweck der Implantation durch einen Katheter zusammengefaltet werden kann und sich nach der Implantation, d.h. nach dem Ausstoßen aus dem Katheter selbststätig auffaltet, insbesondere unter Einwirkung der Körperwärme. Eine solche Faltbarkeit kann nicht nur durch Formgedächtnislegierungen erzielt werden, sondern auch durch geeignete biokompatible Federstahllegierungen.

Das wenigstens eine erfindungsgemäße Anlageband kann in einer möglichen, eine Federelastizität bereitstellenden Ausführung auch durch einen spiralförmig gewundenen Draht ausgebildet sein, der durch seine Windung einen in Erstreckungsrichtung des Anlagebandes liegenden hohlen Kanal. Ein solches innen offenes Anlageband kann auch durch ein innen hohles Drahtgeflecht gebildet werden. Dieser gewundene Draht, bzw. das Drahtgeflecht kann zusätzlich in ein biokompatibles Material eingebettet sein, z.B. in ein Silikon, insbesondere ein Material, welches auch den Verschlußkörper umgibt oder aus welchem der Verschlußkörper zumindets z.T. gebildet ist.

Der Draht kann selbst aus einer Formgedächtnislegierung, z.B. Nitinol ausgebildet sein. Alternativ oder ergänzend kann es auch vorgesehen sein, im Inneren des spiralförmig gewundenen Drahtes / Drahtgeflechtes, der/das also einen Kanal bildet, einen weiteren Draht zu führen, z.B. einen aus einer Formgedächtnislegierung wie Nitinol.

Wenigstens ein Anlageband, bzw. eine zuvor beschriebene Käfigkonstruktion ist zumindest an einem der Enden des Verschlußkörpers einer erfindungsgemäßen Vorrichtung angeordnet, bevorzugt jedoch an beiden Enden, also einen unteren und oberen Ende des Verschlußkörpers.

Bei einer Vorrichtung zu Behebung einer Insuffizienz der Mitralklappe oder auch der Trikuspidalklappe kann somit ein Anlageband oder können mehrere Anlagebandarme wenigstens eine Schlaufe ausbilden, die im Atrium des Herzens angeordnet ist und ein Anlageband bzw. mehrere Anlagebandarme können wenigstens eine Schlaufe ausbilden, die in einem Ventrikel angeordnet ist. Der Verschlußkörper befindet sich zwischen den Schlaufenpaaren / Käfigpaaren und wird so im Durchgangsbereich der Herzklappe positioniert und befestigt. Insbesondere wird hierdurch eine große axiale wie auch eine große laterale Positionsgenauigkeit senkrecht zur Blutströmung erzielt.

Um die Federeigenschaften einer gebildeten Schlaufe zu verbessern kann es in einer auf alle Ausführungen anwendbaren Weiterbildung vorgesehen sein, dass in einer Schlaufe des Anlagebandes, insbesondere in derjenigen Schlaufe, die dem Ventrikel zugeordnet ist, wenigstens ein Bogen / Knick angeordnet ist, dessen Spitze zum Verschlußkörper weist.

Bei allen Ausführungen kann es in einer Weiterbildung vorgesehen sein, dass ein Anlageband in / an den sich zwischen den Enden erstreckenden Seitenbereichen des Verschlußkörpers befestigt ist. Das Anlageband bzw. ein Anlagebandarm wird somit bei dieser Ausführung zumindest auf einem Teil seiner Erstreckung längs der Erstreckung des Verschlußkörpers geführt und an / in diesem befestigt. Statt einer Befestigung an / in den Seitenbereichen kann eine Befestigung auch in dem Verschlußkörper, z.B. mittig oder einem gewünschten Ort im inneren Volumen des Verschlußkörpers erfolgen.

Es kann bei vorgenannten Ausführungen auch eine bevorzugte Weiterbildung erzielt werden, bei der durch ein einziges, insbesondere in sich geschlossenes Anlageband eine Schlaufe über dem oberen Ende und eine Schlaufe unter dem unteren Ende des Verschlußkörpers ausgebildet ist, insbesondere wobei die beiden Schlaufen durch die Seitenbereiche des Verschlußelementes oder z.B. mittig durch dieses hindurch ineinander übergehen.

Bei einer Käfigbildung durch mehrere Anlagebandarme können zueinander korrespondierende Arme am unteren und oberen Ende des Verschlußkörpers durch diesen hindurch ineinander übergehen.

Bei einer getrennten Ausführung von oberem und unterem Anlageband können diese auch jeweils an den in Strömungsrichtung liegenden stirnseitigen oberen und unteren Enden befestigt sein.

Bei einer Anwendung zur Behandlung einer Mitralklappeninsuffizienz kann es vorgesehen sein, dass ein Verschlußkörper einen im Wesentlichen sichelförmigen Querschnitt senkrecht zur Strömungsrichtung des Blutes aufweist. Bei dieser Ausführung kann das Anlageband, insbesondere das einzige Anlageband in den Sichelspitzen dieses Querschnittes liegen.

In analoger Weise können bei einer Anwendung zur Behandlung einer Trikuspidalklappeninsuffizienz, bei der der Verschlußkörper senkrecht zur Strömungsrichtung einem im Wesentlichen dreiarmig sternförmigen Querschnitt aufweist, die Anlagebandarme in den Armspitzen liegen. Eine Käfigform der Anlagebandarme wird daher bevorzugt bei einer solchen Anwendung zum Einsatz kommen.

Bei sämtlichen Anwendung kann auch eine Ausführung vorgesehen sein, bei der wenigstens eine der Schlaufen, insbesondere die dem Atrium zugeordnete(n) Schlaufe(n) eines der Enden des Verschlußkörpers zur Herzwand konvex ausgebildet ist (sind) und wenigstens eine der Schlaufen, insbesondere die dem Ventrikel zugeordnet ist (sind), eine zum Verschlußkörper distale, zur Herzwand gerichtete Spitze bildet, insbesondere die im Herzmuskel verankerbar ist.

So wird erfindungsgemäß erreicht, dass das obere Ende des Verschlußkörpers durch Anlage der dem Atrium zugeordneten Schlaufe an der inneren Herzwand befestigt wird, das untere Ende jedoch im Myokard fixiert wird, im Wesentlichen, wie es bereits im Stand der Technik bekannt ist. So wird zumindest zu beiden Enden des Verschlußkörpers eine Befestigung im Herzen erzielt. Durch die Schlaufe im Atrium wird zudem eine Bewegung des Verschlusskörpers mit dem Blutstrom aus der Klappenebene hinaus in den Ventrikel verhindert.

Um die Befestigung an der Herzinnenwandung noch zu verbessern, kann es bei allen möglichen Ausführungen vorgesehen sein, dass an den Außenseiten des wenigstens einen Anlagebandes Widerhaken oder Noppen angeordnet sind. Mit diesen Widerhaken kann sich ein Anlageband in der Herzwandung verkrallen bzw. über die Noppen eine Positionsänderung verhindern.

Es kann auch in einer mit allen Ausführungen kombinierbaren Weiterbildung vorgesehen sein, dass ein Anlageband in Bereichen seiner Erstreckung, in denen es an die innere Herzwand anlegbar ist, einen gegenüber anderen Bereichen (insbesondere am Ort des Verschlußkörpers) abgeflachten und verbreiterten Querschnitt aufweist. So wird in den Anlagebereichen eine Flächenvergrößerung erzielt und hierdurch die Reizung der Herzwand verringert.

In einer bevorzugten und ebenso mit allen Ausführungen kombinierbaren Weiterbildung kann es vorgesehen sein, dass nicht nur das wenigstens eine Anlageband bzw. Anlagebandarme aus einem kollabierten oder gefalteten Zustand auffaltbar ausgebildet ist, sondern ebenso der Verschlußkörper durch innere Kraftbeaufschlagung aus einem gefalteten / kollabierten Zustand entfaltbar ist.

So kann die gesamte erfindungsgemäße Vorrichtung als ein Bauteil durch einen Katheter oder über eine Schleuse in das Herz eingeführt werden, wobei die Vorrichtung innerhalb des Katheters / Schleuse in einem kollabierten / gefalteten Zustand vorliegt und nach Freigabe aus dem Katheter / Schleuse entfaltet werden kann. Der Vorgang der Entfaltung kann dabei z.B. über die elastische Struktur der Anlagebänder erreicht werden.

Eine innere Kraftbeaufschlagung kann z.B. dadurch erfolgen, dass ein Fluid in den Verschlußkörper einpumpbar ist, ein aufquellendes Medium im Verschlußkörper vorgesehen ist oder der Verschlußkörper aus einem solchen besteht bzw. dieses umfasst oder auch mittels der Federkraft wenigstens eines Anlagebandes, das durch den Verschlußkörper hindurchgeführt ist, insbesondere durch dessen Seitenbereiche.

In einer Weiterbildung kann der Verschlußkörper eine zwischen den oberen und unteren Enden angeordnete Querschnittsverjüngung aufweisen. Auf diese Weise kann der Verschlußkörper strömungsgünstig angepasst werden. Dabei, aber auch unabhängig von vorgenannter Ausführung kann es vorgesehen sein, dass der Verschlußkörper, insbesondere zumindest in der Ebene der Herzklappe, an die konkrete zuvor messtechnisch erfasste Form eines Spaltes zwischen den Klappensegeln einer geschlossenen Herzklappe eines Patienten angepasst ist.

Hierfür kann z.B. der Spalt in der geschlossenen Herzklappe radiologisch, sonographisch oder videographisch vermessen werden, um sodann den Verschlußkörper anhand der erfassten Messwerte patientenspezifisch zu fertigen.

Unabhängig von den zuvor beschriebenen Ausführungen kann der Verschlußkörper zumindest auf seiner Oberfläche aus einem quellenden biokompatiblem Material, insbesondere einem Hydrogel ausgebildet sein. Hierdurch wird eine besonders gute Dichtigkeit zwischen den Klappensegeln und dem Verschlußkörper im geschlossenen Zustand der Herzklappe erzielt.

Als weiteres, eine gute Dichtigkeit gewährleistendes Material kann z.B. auch ein biokompatibles Silikon für den Verschlußkörper gewählt werden. Der Verschlußkörper kann einen Überzug aus einem solchen Silikon aufweisen oder auch vollständig aus diesem gefertigt sein.

Eine Ausführung kann auch vorsehen, im Verschlußkörper ein formgebendes Drahtgeflecht anzuordnen, das mit dem Silikon oder einem anderen biokompatiblem Material umgossen / umspritzt ist. Insbesondere kann hier vorgesehen sein, dass das genannte biokompatible Material / Silikon auch das wenigstens eine Anlageband umgibt, sich hierdurch sodann also ein gesamter Überzug der erfindungsgemäßen Vorrichtung mit dem biokompatiblem Material ergibt.

In einer mit allen anderen Ausführungen kombinierbaren Weiterbildung kann es auch vorgesehen sein, dass zumindest der Verschlußkörper, ggfs. auch das wenigstens eine Anlageband eine hydrophile Oberflächenbeschichtung oder -textur aufweist. Ebenso kann die Oberfläche des Verschlußkörpers und/oder des wenigstens einen Anlagebandes derart ausgebildet sein, z.B. durch Beschichtung, dass eine Endothelialisierung gefördert wird.

Besonders durch vorgenannte oder andere geeignete Beschichtungen oder Texturen kann erzielt werden, dass bei dem wiederholten Anlegen der Klappen gegen den Verschlusskörper keine Schädigung der Klappen erfolgt.

In einer weiterhin möglichen und mit allen Ausführungen kombinierbaren Weiterbildung kann es vorgesehen sein, dass am Verschlusskörper, insbesondere an zumindest einer Seite, bevorzugt an zwei gegenüberliegenden Seiten des Verschlußkörpers ein bewegliches, insbesondere durch den Blutstrom bewegliches Klappenelement angeordnet ist. Ein solches Klappenelement kann bevorzugt derart ausgebildet sein, dass es den Querschnitt des Verschlusskörpers bei der natürlichen, gewünschten Strömungsrichtung des Blutes (z.B. vom Atrium ins Ventrikel) vermindert, z.B. dadurch, dass sich das Klappenelement durch den wirkenden Blutstrom oder eine Kraftunterstützung an den Verschlusskörper anlegt und dass es den Querschnitt des Verschlusskörpers bei einer Strömung des Blutes entgegen der vorgenannten Strömungsrichtung, also bei einer Strömungsumkehr vergrößert, z.B. dadurch, dass das Klappenelement durch das strömende Blut von dem Verschlusskörper abgehoben wird oder hierdurch entfaltet wird. Ein solches Klappenelement kann sich z.B. über die gesamte Breite eines Verschlußkörpers erstrecken, bei einem sichelförmigen Verschlußkörper z.B. zwischen den Sichelspitzen.

Bei dieser Ausführung kann eine natürliche Herzklappe mit wenigstens einem beweglichen Klappenelement zusammenwirken und ein sicheres Verschließen des Spaltes in der Herzklappe bewirken.

Ausführungsbeispiele der Erfindung werden nachfolgend beschrieben. Es zeigen:
- Figur 1:: Eine Ausführung mit einem konvex schlaufenförmigen Anlageband am oberen und unteren Ende eines Verschlußkörpers für die Mitralklappe
- Figur 2:: Eine Ausführung mit einem oberen konvex schlaufenförmigen Anlageband und einer unteren spitz zulaufenden Schlaufe des Anlagebandes
- Figur 3:: Eine Ausführung für die Trikuspidalklappe mit jede drei Anlagebandarmen am oberen und unteren Ende
- Figur 4:: Eine Ausführung mit einem konvex schlaufenförmigen Anlageband am oberen Ende eines Verschlusskörpers für die Mitralklappe
- Figur 5:: Eine Ausführung für die Mitralklappe mit der Verwendung eines Klappenelementes am Verschlusskörper
- Figur 6:: Sichelförmige Querschnitte des Verschlußkörpers für eine Mitralklappeninsuffizienz.

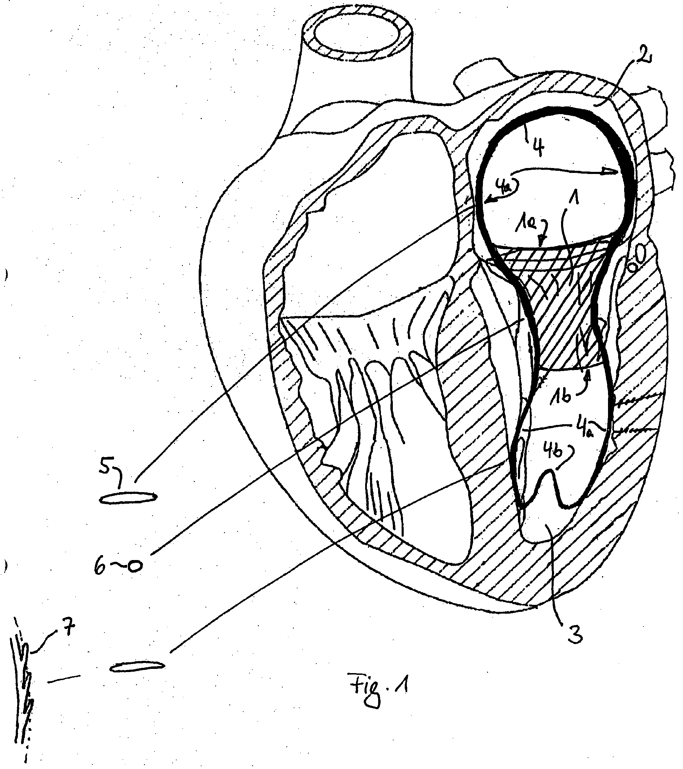

Die Figur 1 zeigt eine erste Ausführungsform der erfindungsgemäßen Vorrichtung zur Behandlung einer Mitralklappeninsuffizienz. Der Verschlußkörper 1 ist zwischen Atrium 2 und Ventrikel 3 innerhalb der Mitralklappe positioniert, d.h. von der Mitralklappe umgeben. Die Querschnittsform des Verschlußkörpers 1 ist bevorzugt an die Querschnittsform des verbleibenden Spaltes der geschlossenen Mitralklappe angepasst.

Am oberen Ende 1a des Verschlußkörpers 1 befindet sich ein schlaufenförmig konvex zur Herzwand geformtes Anlageband 4, das sich vom Verschlußkörper 1 weg erstreckt und in einer konvex bogenförmigen Schlaufe zum Verschlußkörper zurückgeführt und in dessen seitlichen Bereichen am Verschlußkörper 1 befestigt ist.

Das Anlageband 4 kontaktiert die Herzwandung von innen in lateralen Bereichen 4a. In diesem Bereichen 4a kann das Anlageband einen abgeflachten Querschnitt 5 aufweisen, wohingegen in den übrigen Bereichen, insbesondere den Befestigungsbereichen am Verschlußkörper der Querschnitt 6 des Anlagebandes 4 rund sein kann.

Am unteren Ende 1 b des Verschlußkörpers ist ebenso ein Anlageband 4 vorgesehen, das einen schlaufenförmigen gebogenen Verlauf vollzieht, grundsätzlich vergleichbar mit der oberen Schlaufe. Die untere Schlaufe weist hier jedoch einen Bogen auf, dessen Spitze zum Verschlußkörper weist. Dieser Bogen kann die laterale Federelastizität zwischen den Bereichen 4a des unteren Anlagebandes 4 unterstützen.

In dieser Ausführung sind unteres und oberes Anlageband 4 identisch. Die Schlaufen am oberen und unteren Ende 1a/1b des Verschlußkörpers 1 gehen ineinander über, dadurch, dass das Anlageband in den seitlichen Bereichen des Verschlußkörpers durch diesen hindurchgeführt oder an diesen vorbei geführt ist und am Verschlußkörper befestigt ist.

Die Figur 1 zeigt weiterhin, dass in dieser Ausführung die Außenseite des Anlagebandes 4, insbesondere zumindest in den abgeflachten Kontaktbereichen 4a Widerhaken oder Noppen 7 aufweist.

Bei der in der Figur 1 gezeigten Ausführung erfolgt die Befestigung im Herzen nur durch eine Kontaktierung des Anlagebandes 4 an der inneren Herzwand.

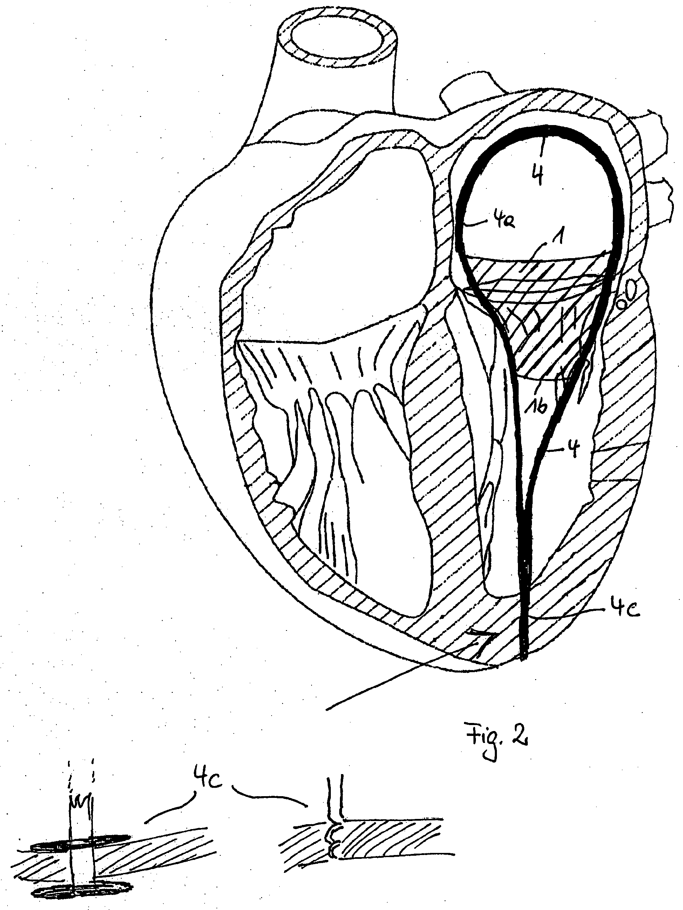

Demgegenüber unterscheidet sich die Figur 2, bei der das Anlageband 4 am unteren Ende 1 b des Verschlußkörpers 1 in einer am distalen Ende spitz zulaufenden Schlaufe geführt ist. Das zum Verschlußkörper 1 distale Schlaufenende 4c kann einen Befestigungsbereich aufweisen, um eine Verankerung im Myokard herzustellen, z.B. durch ein Schraubengewinde oder durch Ankerplatten die das Myokard umschließen.

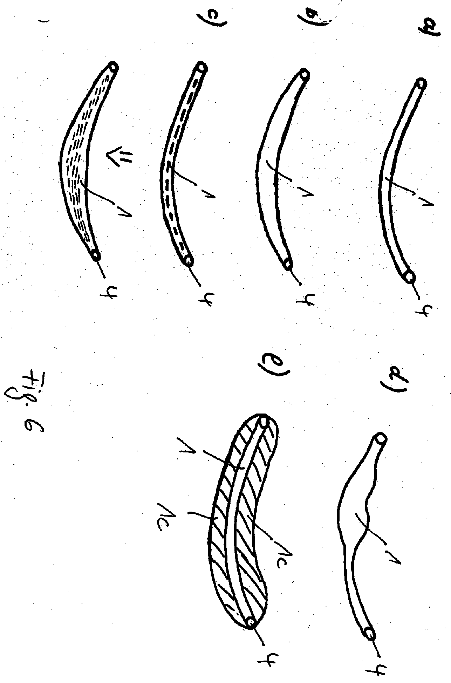

Bei den Figuren 1 und 2 ist der Verschlußkörper 1 an den Spalt der Mitralklappe angepasst und weist daher grundsätzlich im Querschnitt senkrecht zur Fließrichtung des Blutes eine Sichelform auf, wie es Figur 6 zeigt. Das Anlageband 4 ist hier bevorzugt durch die jeweilige Sichelspitze hindurchgeführt.

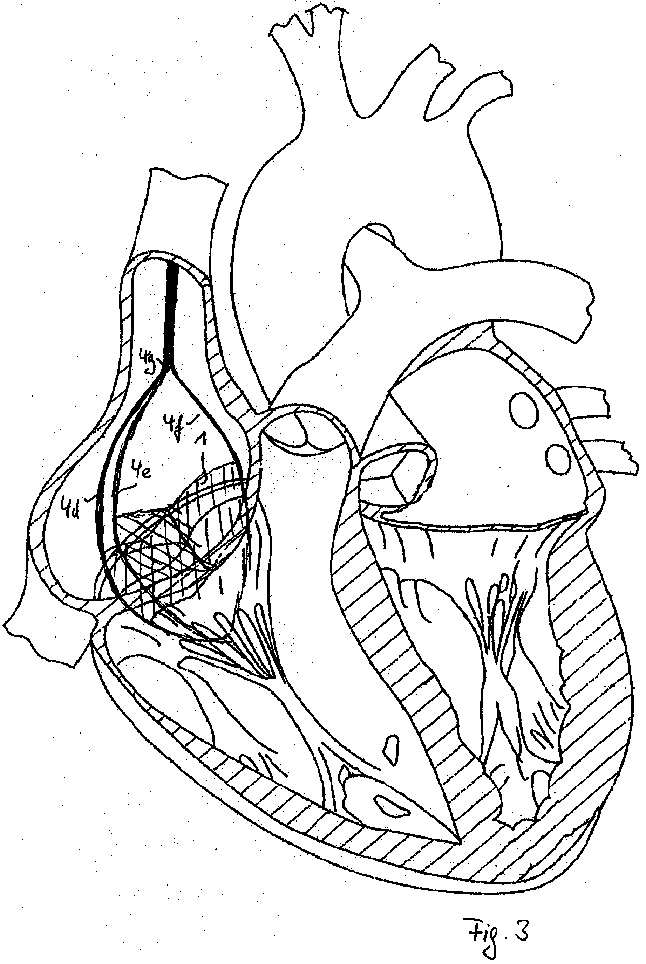

Die Figur 3 zeigt eine Ausführung zur Behandlung einer Insuffizienz der Trikuspidalklappe. Hier ist am sternförmig mit drei Armen ausgebildeten Verschlußkörper 1 am oberen und am unteren Ende ein Anlageband mit drei Anlagebandarmen angeordnet, wobei die Arme 4d,e,f an einem gemeinsamen Ort 4g zusammengeführt sind. Die Arme 4d, e, f bilden somit quasi einen Käfig, der das obere und untere Ende des Verschlußkörpers überspannt.

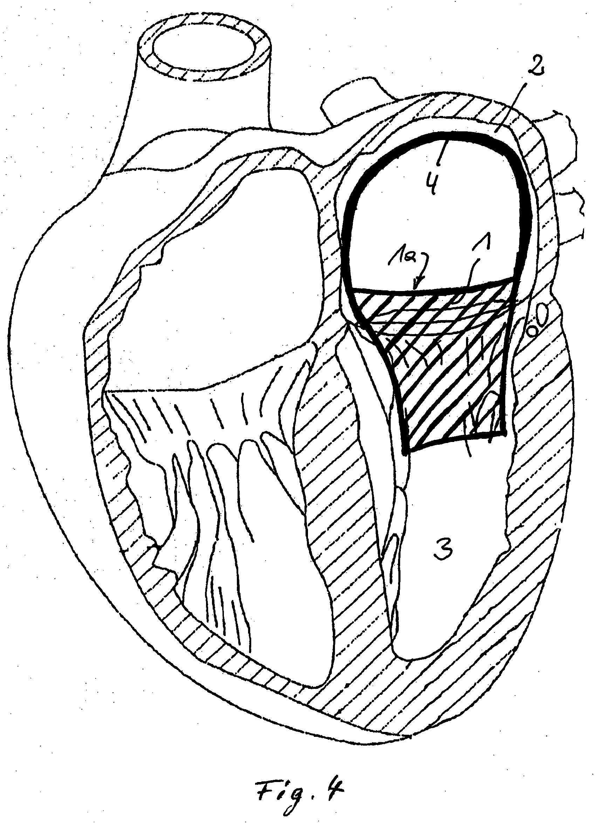

Bei der in Figur 4 gezeigten Ausführung wird die Behandlung einer Insuffizienz der Mitralklappe durch einen Verschlusskörper 1 erreicht, der mit einem konvex schlaufenförmigen Anlageband 4 nur am oberen Ende eines Verschlusskörpers 1 für die Mitralklappe versehen ist.

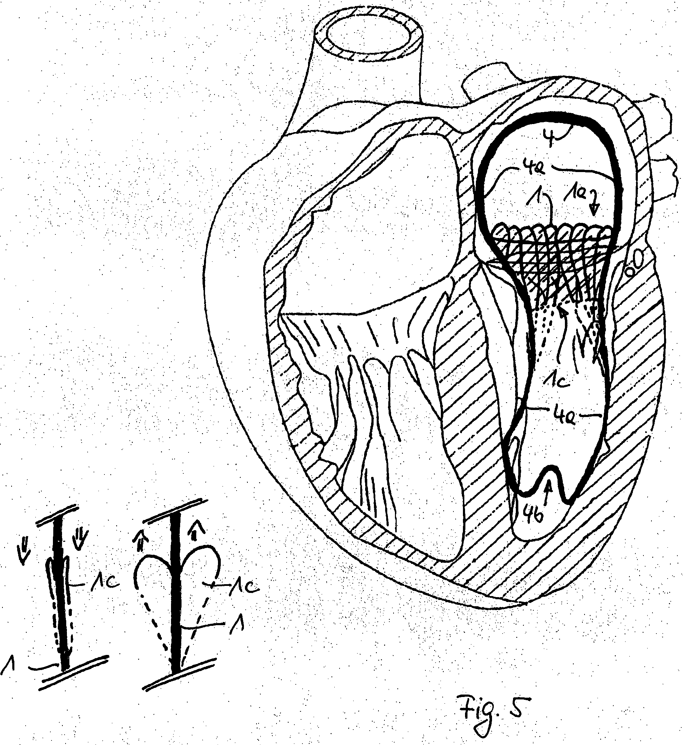

Bei der in Figur 5 gezeigten Ausführung wird die Behandlung einer Insuffizienz der Mitralklappe entsprechend der in Figur 1 dargelegten Ausführung erreicht. Anstelle eines reinen Verschlusskörpers 1 wird der Rückstrom zusätzlich durch ein Klappenelement 1 c erreicht, das beweglich am Verschlußkörper 1 angeordnet ist. Das am Verschlusskörper 1 anliegende Klappenelement 1 c stellt sich auf oder entfaltet sich mit Flussumkehr und verhindert darüber den Rückstrom des Blutes aus dem Ventrikel 3 in das Atrium 2.

Bei allen hier gezeigten Ausführungen kann die erfindungsgemäße Vorrichtung entfaltbar sein, um z.B. aus einem Katheter / Schleuse heraus appliziert zu werden. Wie die Figur 6a und b zeigt, kann der Verschlußkörper eine Hohlform sein, deren inneres Volumen gefüllt werden kann, um den Verschlußkörper zu entfalten.

Gemäß Figur 6c kann der Verschlußkörper 1 auch mit einem aufquellbaren Material 8 gefüllt sein, so dass dieser automatisch durch Blutkontakt aufquillt und seine nötige Form annimmt. Auch ist eine individuell an die Klappenmorphologie angepasste Form des Verschlusskörpers 1 gemäß Figur 6d möglich. Die Form des Klappenspaltes kann z.B. messtechnisch erfasst und der Verschlußkörper 1 anhand der erfassten Daten gefertigt werden.

Ebenfalls ist die Erweiterung des Verschlußkörpers mit wenigstens einem Klappenelement 1 c, hier beidseitig je einem Klappenelement 1 c gemäß Figur 6e möglich, welches jeweils bei Flussumkehr den Querschnitt des Verschlusskörpers 1 erweitert und den Rückstrom des Blutes verhindert.

## Patentansprüche

1. Implantierbare Vorrichtung zur Verbesserung oder Behebung einer Insuffizienz der Mitralklappe oder der Trikuspidalklappe umfassend einen Verschlußkörper (1), der im Durchgangsbereich einer Herzklappe zwischen einem Atrium (2) und einem Ventrikel (3) des Herzens positionierbar ist und mit dem der im geschlossenen Zustand einer Herzklappe verbleibende Spalt ausfüllbar und hierdurch verschließbar ist oder zumindest verkleinerbar ist, so dass der Herzklappe ihre Funktion im geschlossenen Zustand zurückgebbar ist und der Verschlußkörper durch innere Kraftbeaufschlagung aus einem gefalteten / kollabierten Zustand entfaltbar ist, wobei der Verschlußkörper ein oberes, stromaufwärtiges Ende (1 a) und ein unteres, stromabwärtiges Ende (1 b) aufweist, **dadurch gekennzeichnet, dass** wenigstens ein Anlageband wenigstens eine Schlaufe ausbildet, die im Atrium des Herzen positionierbar ist und wenigstens ein Anlageband wenigstens eine Schlaufe ausbildet, die im Ventrikel positionierbar ist, wobei sich das jeweilige Anlageband von dem Verschlußkörper (1) weg erstreckt und in wenigstens einer Schlaufe zu dem Verschlußkörper (1) zurückgeführt ist und zumindest entlang eines Teils seiner Erstreckung an die innere Herzwand anlegbar ist und wobei der Verschlußkörper sich zwischen den Schlaufenpaaren befindet und so im Durchgangsbereich der Herzklappe positionierbar und befestigbar ist.

2. Vorrichtung nach Anspruch 1, **dadurch gekennzeichnet, dass** die dem Atrium zugeordnete(n) Schlaufe(n) eines der Enden des Verschlußkörpers zur Herzwand konvex ausgebildet ist (sind) und die dem Ventrikel zugeordnete(n) Schlaufe(n) eine zum Verschlußkörper distale, zur Herzwand gerichtete Spitze bildet, die im Herzmuskel verankerbar ist.

3. Vorrichtung nach einem der vorherigen Ansprüche, **dadurch gekennzeichnet, dass** durch ein einziges in sich geschlossenes Anlageband (4) eine Schlaufe über dem oberen Ende (1a) und eine Schlaufe unter dem unteren Ende (1 b) des Verschlußkörpers (1) ausgebildet ist.

4. Vorrichtung nach Anspruch 3, **dadurch gekennzeichnet, dass** die beiden Schlaufen durch die Seitenbereiche oder durch das innere Volumen des Verschlußelementes (1) ineinander übergehen.

5. Vorrichtung nach einem der vorherigen Ansprüche, **dadurch gekennzeichnet, dass** ein Anlageband (4) durch einen in Erstreckungsrichtung des Anlagebandes (4), einen Kanal bildenden, spiralförmig gewundenen Draht oder ein kanalbildendes Drahtgeflecht ausgebildet ist, insbesondere wobei im Inneren des gebildeten Kanales ein weiterer Draht, insbesondere aus einer Formgedächtnislegierung verläuft.

6. Vorrichtung nach einem der vorherigen Ansprüche, **dadurch gekennzeichnet, dass** in einer Schlaufe des Anlagebandes (4), die dem Ventrikel (3) zugeordnet ist, wenigstens ein Bogen / Knick (4b) angeordnet ist, dessen Spitze zum Verschlußkörper (1) weist.

7. Vorrichtung nach einem der vorherigen Ansprüche, **dadurch gekennzeichnet, dass** ein Anlageband (4) in / an den sich zwischen den Enden erstreckenden Seitenbereichen des Verschlußkörpers befestigt ist, insbesondere denjenigen Seitenbereichen, die bei einem sichelförmigen Querschnitt des Verschlußkörpers (1) senkrecht zur Strömungsrichtung in den Sichelspitzen liegen.

8. Vorrichtung nach einem der vorherigen Ansprüche, **dadurch gekennzeichnet, dass** wenigstens ein Anlageband (4) entlang seines Erstreckungsweges in wenigstens zwei Anlagebandabschnitte (4d, 4e, 4f) aufzweigt, insbesondere an einem Ort (4g) maximaler Entfernung des Aufzweigungsortes vom Verschlußkörper (1).

9. Vorrichtung nach einem der vorherigen Ansprüche, **dadurch gekennzeichnet, dass** vom Verschlußkörper ausgehende mehrere Anlagebandarme sich auf einem schlaufenförmigen oder bogenförmigen Weg erstrecken und in einer gemeinsamen Verbindungstelle treffen und dort zusammengeführt sind, wodurch ein Käfig aus mehreren Anlagebandarmen ausgebildet ist, die mit einem der Enden am Verschlußkörper und mit den anderen Enden untereinander befestigt sind.

10. Vorrichtung nach Anspruch 9, **dadurch gekennzeichnet, dass** bei einer Käfigbildung durch mehrere Anlagebandarme zueinander korrespondierende Arme am unteren und oberen Ende des Verschlußkörpers durch diesen hindurch ineinander übergehen.

11. Vorrichtung nach einem der vorherigen Ansprüche, **dadurch gekennzeichnet, dass** ein Anlageband (4) in Bereichen (5) seiner Erstreckung, in denen es an die innere Herzwand anlegbar ist, einen gegenüber anderen Bereichen (6) abgeflachten und verbreiterten Querschnitt aufweist.

12. Vorrichtung nach einem der vorherigen Ansprüche, **dadurch gekennzeichnet, dass** der Verschlußkörper (1) eine zwischen den oberen und unteren Enden (1a, 1b) angeordnete Querschnittsverjüngung aufweist.

13. Vorrichtung nach einem der vorherigen Ansprüche, **dadurch gekennzeichnet, dass** der Verschlußkörper (1), insbesondere zumindest in der Ebene der Herzklappe, an die konkrete zuvor messtechnisch erfasste Form eines Spaltes zwischen den Segeln einer geschlossenen Herzklappe eines Patienten angepasst ist.

14. Vorrichtung nach einem der vorherigen Ansprüche, **dadurch gekennzeichnet, dass** der Verschlußkörper (1) zumindest auf seiner Oberfläche aus einem quellenden biokompatiblem Material, insbesondere einem Hydrogel ausgebildet ist oder der Verschlußkörper (1) eine hydrophile Oberfläche oder eine die Endothelialisierung fördernde Beschichtung / Textur der Oberfläche aufweist.

15. Vorrichtung nach einem der vorherigen Ansprüche, **dadurch gekennzeichnet, dass** am Verschlusskörper (1), insbesondere an zumindest einer Seite, bevorzugt an zwei gegenüberliegenden Seiten des Verschlußkörpers ein bewegliches Klappenelement (1 c) angeordnet ist.
